# EUROPÄISCHE PATENTSCHRIFT

(11) **EP 0 850 052 B1**
(45) Veröffentlichungstag und Bekanntmachung des Hinweises auf die Patenterteilung: **12.12.2001**
(21) Anmeldenummer: 96932512.5
(22) Anmeldetag: 12.09.1996
(51) Int. Cl.: A61K 9/70

(54) **TACRIN/SELEGILIN-PFLASTER**
TACRINE/SELEGILINE PATCH
TIMBRE A LA TACRINE ET A LA SELEGILINE

(30) Priorität: 12.09.1995 DE 19533772
(43) Veröffentlichungstag der Anmeldung: 01.07.1998
(73) Patentinhaber: HEXAL AG, D-83607 Holzkirchen (DE)
(72) Erfinder: SENDL-LANG, Anna, D-83607 Holzkirchen (DE); FISCHER, Wilfried, D-83607 Holzkirchen (DE)
(74) Vertreter: Boeters, Hans Dietrich, Dr.
(86) Internationale Anmeldenummer: EP9604010
(87) Internationale Veröffentlichungsnummer: WO9709969

(56) Entgegenhaltungen:
- EP-A- 0 241 809
- EP-A- 0 332 147
- EP-A- 0 499 662
- EP-A- 0 515 302
- WO-A-89/09051

## Beschreibung

Tetrahydroaminoacridin (Tacrin) wird bei der Alzheimer-Krankheit als ein zentral wirksamer Cholinesterasehemmer verwendet. Seine Cholinesterasehemmung hält etwa 15 h an, also deutlich länger als die Halbwertszeit von Tacrin, die im Bereich von 1,5 bis 3,5 h liegt. Die tägliche Dosisempfehlung variiert zwischen 30 und 160 mg, wobei der therapeutische Blutspiegel im Bereich von 5 bis 70 ng/ml und die Bioverfügbarkeit im Bereich von 10 bis 30 % liegt. Bei Patienten, die an der Alzheimer-Krankheit leiden und über eine längere Zeit mit mehreren Tagesdosen an Tacrin behandelt werden, läßt sich eine klinische Verbesserung des Gedächtnisses und der funktionalen Autonomie beobachten.

Tacrin wird oral in Form des Hydrochlorids eingesetzt (Cognex^{R}). Da die orale Gabe von Tacrin wegen seiner kurzen Eliminationshalbwertszeit in multiplen Dosen erfolgen muß, andererseits die Freisetzung des Wirkstoffs über eine lange Zeit konstant bleiben sollte, würde eine transdermale Gabe eine effektivere Arzneimitteltherapie darstellen. Ein zusätzliches Problem stellen die stark ausgeprägte First-Pass-Metabolisierung und die stark schwankende Pharmakokinetik von Tacrin dar, die eine schlechte Einstellbarkeit der Patienten, das Auftreten von Blutplasmaspitzen sowie eine Erhöhung der Nebenwirkungen und der Toxizität vor allem auf die Leber zur Folge haben. Der hepatotoxische Effekt des Tacrins tritt bei etwa der Hälfte der Patienten auf, wobei ein Anstieg der ALT-Werte (Alaninaminotransferase) bis zum 20-fachen der oberen normalen Grenze beobachtet wird. Durch eine kontinuierliche transdermale Gabe von Tacrin könnte es zu konstanteren und niedrigeren therapeutisch wirksamen Plasmaspiegeln und somit zu geringeren Nebenwirkungen vor allem auf die Leber kommen, zumal der hohe First-Pass-Effekt und die geringe Bioverfügbarkeit wegfallen sollten.

EP-B-0 332 147 beschreibt ein transdermales System mit einem Gehalt an
(a) unter anderem Tetrahydroaminoacridin oder einem pharmazeutisch annehmbaren Salz dieser Verbindung als Wirkstoff,
(b) einem Propylenglycoldiester von Caprylsäure und Caprinsäure (Migliol) und
(c) Kieselsäure als Geliermittel.
   Migliol erhöht in diesem transdermalen System den Substanzflux durch Mäusehaut im Vergleich zu einer Formulierung ohne Migliol. Fakultativ kann ein Permeationsbeschleuniger in Form eines Lösungsmittels zugegeben werden, beispielsweise Alkohol. Alkohol reizt jedoch die Haut, trocknet sie aus und schädigt sie. Der Einsatz von Ethanol ist kritisch zu beurteilen, da er die natürliche Lipidschicht der Haut stört und sie austrocknet und reizt. Hinzu kommt, daß nach Aussage von EP-B-0 499 662 die Freisetzungskontrolle des Wirkstoffs beim Pflaster gemäß EP-B-0 332 147 nicht optimal ist.

So wird nach der EP-B-0 499 662 ein zweilagiges Verbundlaminat als transdermales System vorgesehen, das aus
A) einer Schicht I mit einer Komponente A, die aus einem Lösungsmittel und einem aktiven Wirkstoff in einem teilweise ausgehärteten Elastomeren besteht, und
B) einer Schicht II auf der Schicht I besteht, die eine Komponente B umfaßt, die aus einem Lösungsmittel und einem Wirkstoff in einem makroporösen Formkörper mit einer Porengröße von 10 bis 100 µm besteht.

Als Beispiel für einen Wirkstoff wird unter anderem 1,2,3,4-Tetrahydro-9-acridinamin (THA = Tacrin) genannt. Als Lösungsmittel für die Komponenten A und B werden Alkohole vorgeschlagen, beispielsweise Ethanol. Durch Ethanol wird jedoch wiederum die Haut ausgetrocknet, gereizt und geschädigt.

Zudem ist das aus EP-B-0 499 662 bekannte schwammartige transdermale System dick und unflexibel und somit bei Anwendung durch den Patienten nicht sehr praktikabel, da das System sich durch seine Höhe exponiert, leicht ungewollt entfernbar ist und Körperbewegungen nicht sehr gut mitmacht.

Das aus EP-B-0 499 662 bekannte transdermale System wird dadurch hergestellt,-daß man
(a) einen Wirkstoff mit einem Lösungsmittel und einer härtbaren Elastomerkomponente mischt,
(b) die erhaltene Komponente A zu einer teilgehärteten Schicht I gießt,
(c) einen Wirkstoff in einem Lösungsmittel löst,
(d) die erhaltene Lösung in einen makroporösen Formkörper einführt und Komponente B erhält, die die Schicht II bildet,
(e) Schicht II auf Schicht I laminiert und gegebenenfalls das anfallende Laminat zu Pflastern gewünschter Größe zuschneidet.

Diese Herstellung ist schwierig nachvollziehbar, kompliziert und führt außerdem zu wenig reproduzierbarem Blutspiegel, möglicherweise durch die schlecht kontrollierbare Teilhärtung des Elastomeren. Wenn außerdem bei diesem sehr kostenaufwendigen Verfahren über mehrere Minuten eine erhöhte Temperatur von beispielsweise 160 °C angewendet wird, kann dadurch der Wirkstoff abgebaut werden. Neben der Frage, wie eine Aluminiumfolie auf den makroporösen Schwamm aufzulaminieren ist, bleibt die Frage offen, wie das transdermale System am Patienten appliziert werden kann, da offensichtlich kein klebendes Element vorgesehen ist.

Nach Auterhoff, Knabe & Höltge, Lehrbuch der Pharmazeutischen Chemie, 12. Auflage, Seite 484 ist (R)(-)-N-Methyl-N-(1-phenyl-2-propyl)-2-propinylamin (= Selegilin = Deprenyl, im Handel als Movergan^{R}) ein Antiparkinsonmittel mit dopaminerger Wirkung. EP-A-0 241 809 beschreibt ein Pflaster (Spalte 5 Zeile 15) mit einem Gehalt an Selegilin, wobei dieses Pflaster in Kombination mit einem weiteren Pflaster mit einem Gehalt an Amantadin eingesetzt wird. Ferner ist aus EP-B-0 404 807 ein Pflaster (Seite 3 Zeile 57) als TTS mit Selegilin als einzigem Wirkstoff bekannt, wobei der Wirkstoff aus einer Matrix heraus die Haut penetriert. DE-9 523 299 beschreibt ein weiteres transdermales System (TTS) mit einer Abdeckfolie, einer Schicht mit einem Gehalt an Selegilin als Wirkstoff, einer Membran, einer selbstklebenden Schicht und einer entfernbaren Schutzschicht.

Gegenüber diesem Stand der Technik ist es eine Aufgabe der Erfindung, ein Pflaster zur transdermalen Applikation vorzusehen, mit dem sich die Alzheimer-Krankheit noch besser bekämpfen läßt. Eine weitere Aufgabe der Erfindung besteht darin, die Hautpermeation der Wirkstoffe und insbesondere von Tacrin zu erhöhen.

Dazu wird gemäß einer ersten Ausführungsform der Erfindung ein transdermales System beziehungsweise Pflaster mit einer äußeren Abdeckschicht (outer covering bzw. backing layer), einer selbstklebenden Matrix (self-adhesive matrix) und einer entfernbaren Schutzschicht (protective liner bzw. release liner) vorgesehen, bei dem die Matrix
- Tacrin mit Selegilin (gegebenenfalls in Form ihrer pharmazeutisch verträglichen Salze) als Wirkstoff neben
- einem hydrophilen schwerflüchtigen Lösungsmittel und/oder
- einem lipophilen schwerflüchtigen Lösungsmittel enthält.

In Hinblick auf die Anstrengungen, die nach EP-B-0 499 662 unternommen wurden, um ein Pflaster mit Tacringehalt vorzusehen, konnte nicht erwartet werden, daß sich mit einem erfindungsgemäßen Pflaster befriedigende Ergebnisse erreichen lassen würden. Das erfindungsgemäße Pflaster läßt sich darüberhinaus kostengünstig, reproduzierbar und ohne größere Wärmebelastung herstellen.

Das erfindungsgemäße Pflaster ist dünn, hautverträglich, flexibel und sehr gut auf die Haut applizierbar.

Das erfindungsgemäße Pflaster unterscheidet sich von einem Pflaster nach EP-B-0 332 147 dadurch, daß der bzw. die Wirkstoffe nicht aus einem Gel unter Verwendung von Kieselsäure als Gelierungsmittel austreten, vielmehr aus einer selbstklebenden Matrix ohne Gehalt an Kieselsäure. Mit dem erfindungsgemäßen Pflaster kann auch bewußt darauf verzichtet werden, die Wirkstoffe wie bei EP-B-0 499 662 in zwei verschiedenen Schichten vorzusehen, die ein Laminat bilden. Vielmehr können die Wirkstoffe in einer einzigen Schicht des erfindungsgemäßen Pflasters vorgesehen werden, bei der es sich um eine selbstklebende Matrix handelt.

Nachdem für orale Applikation von Tacrin die empfohlene tägliche Dosis im Bereich von 30 bis 160 mg und der therapeutische Blutspiegel im Bereich von 5 bis 70 ng/ml liegen, war nicht zu erwarten, daß ein Pflaster zur transdermalen Applikation mit einer vergleichbaren Wirkstoffmenge beladbar und vergleichbare Blutspiegel erzielbar sein würden.

Das erfindungsgemäße Pflaster kann durch einen Gehalt von bis zu 70 Gew.-% (bezogen auf das Matrixgewicht) an einem Lösungsmittel aus der durch hydrophile schwerflüchtige Lösungsmittel, lipophile schwerflüchtige Lösungsmittel und deren Gemische gebildeten Gruppe gekennzeichnet sein.

Beim erfindungsgemäßen Pflaster können die beiden Wirkstoffe im Gemisch in der Matrix vorliegen. Dabei überrascht, daß sich die Hautpermeation durch die Gegenwart von Selegilin im Vergleich zu einem Pflaster mit Tacrin als alleinigem Wirkstoff erhöhen läßt.

Beim erfindungsgemäßen Pflaster kann die Matrix auch zwei aufeinanderfolgende Matrixschichten aufweisen, wobei die eine Matrixschicht den einen und die andere Matrixschicht den anderen der beiden Wirkstoffe enthält und wobei die der Abdeckschicht (outer covering bzw. backing layer) zugewandte Matrixschicht Selegilin oder eins seiner Salze oder Tacrin oder eins seiner Salze als Wirkstoff enthalten kann. Bei dieser Ausführungsform können die beiden Matrixschichten durch eine Membran getrennt sein, die die Permeation des Wirkstoffs aus der hautferneren Matrixschicht steuert.

Beim erfindungsgemäßen Pflaster kann die Matrix auch zwei oder mehr aneinander angrenzende, nebeneinander in derselben Ebene angeordnete Zonen aufweisen, die jeweils nur einen der beiden Wirkstoffe enthalten. Bei dieser Ausführungsform können die Zonen bahnartig ausgebildet sein.

Der Gehalt an Tacrin und an Selegilin (gegebenenfalls in Form ihrer Salze) kann beim erfindungsgemäßen Pflaster jeweils bis zu 50 Gew.-% betragen (jeweils bezogen auf das Matrixgewicht).

Erfindungsgemäß kann eine Membran, insbesondere eine die Wirkstoffpermeation steuernde Membran vorgesehen werden.

Membran- und Reservoir-Pflaster gehören zum Stand der Technik. Lediglich beispielhaft sei auf WO-A1-89/09 051, WO-A1-94/23 707, EP-B1-0 404 807 und EP-A1-0 406 488 verwiesen.

Für das erfindungsgemäße Pflaster kann man eine Matrix auf Polyacrylatbasis, Silikonbasis oder Polyisobutylenbasis vorsehen. Derartige Matrices sind im Stand der Technik vorgegeben. Lediglich beispielhaft sei für eine Polyacrylat-Matrix auf DE-B-3 933 460, eine Silikon-Matrix auf DE-A-4 339 400 und eine Polyisobutylen-Matrix auf EP-A-0 186 019 verwiesen.

Bei der genannten Ausführungsform des erfindungsgemäßen Pflasters kann das Reservoir durch die Abdeckschicht und die Membran oder durch eine Matrix gebildet werden.

Das Klebeelement kann in Form einer das Reservoir (sofern eine Membran fehlt) oder einer die Membran vollständig oder lediglich an ihrer Peripherie ringförmig abdeckenden Schicht vorgesehen sein. Erfindungsgemäß kann das Klebeelement aus einem druckempfindlichen Klebemittel auf Silikon-Basis bestehen.

Das erfindungsgemäße Pflaster kann ferner durch einen Gehalt an α-Tocopherol oder α-Tocopherol-Derivat gekennzeichnet sein. Durch diesen Zusatz kann man der kritischen Hautverträglichkeit bei der Anwendung von niederen Alkoholen Rechnung tragen.

Für ein erfindungsgemäßes Pflaster kann man als hydrophiles schwerflüchtiges Lösungsmitel beispielsweise Propylenglykol, 1,2-Pentandiol, Glycerin, hydrophiles Cetiol oder Transcutol^{R} verwenden. Propylenglykol und Glycerin werden bereits mit EP-B-0 499 662 vorgeschlagen, allerdings für das vorstehend diskutierte abweichende transdermale System.

Ferner kann man für das erfindungsgemäße Pflaster als lipophiles schwerflüchtiges Lösungsmittel beispielsweise Copherol^{R}, Propylenglykol-Dicaprylat/Dicaprat, wie Migliol^{R}, Isopropylmyristat oder lipophiles Cetiol^{R}, beispielsweise Cetiol HE, verwenden. Isopropylmyristat wird bereits mit EP-B-0 332 147 und Migliol^{R} wird bereits mit EP-B-0 332 147 und EP-B-0 499 662 vorgeschlagen, jeweils jedoch für die vorstehend diskutierten abweichenden transdermalen Systeme.

Beim erfindungsgemäßen Pflaster überrascht besonders, daß eine Kombination von hydrophilem schwerflüchtigen Lösungsmittel mit lipophilem schwerflüchtigen Lösungsmittel zu einer deutlich höheren Hautpermeationsrate als mit jedem Lösungsmittel für sich allein führt.

Auch kann das erfindungsgemäße Pflaster ein viskositätserhöhendes Mittel enthalten, beispielsweise hochdisperses Siliciumdioxid oder Hydroxypropylcellulose.

Nachstehend wird die Erfindung durch Beispiele näher erläutert.

### Beispiel 1: Einschicht-TTS mit Tacrin/Selegilin

Tacrin- und Selegilinbase werden in Ethanol gelöst. Die erhaltene Lösung wird einer Lösung von Acrylatklebstoff (beispielsweise Duro-Tak^{R}, wie Duro-Tak^{R} 326-1753; National Starch & Chemicals) in Ethylacetat und n-Hexan zugefügt und gegebenenfalls mit weiteren Hilfsstoffen gemischt. Danach wird die Lösung mit Hilfe eine Ziehrakels auf eine silikonisierte Polyesterfolie als Abziehfolie (beispielsweise Hostaphan^{R}; Hoechst) mit einer Naßschichtdicke von 450 *µ*m aufgetragen und bei 50 °C 1 h lang getrocknet. Die getrocknete Schicht wird mit einer Polyesterfolie (oder einer anderen geeigneten Abdeckfolie) laminiert. Aus dem Laminat werden mit Hilfe einer Stanze TTS mit Flächen von 10, 20, 30 und 40 cm² ausgestanzt.

### Beispiel 2: Zweischicht-TTS ohne eine die Wirkstoffabgabe steuernde Membran.

Schicht A; Tacrinbase wird (gegebenenfalls zusammen mit Hilfsstoffen) in Ethanol gelöst. Die erhaltene Lösung wird einer Lösung von Acrylatklebstoff (beispielsweise Duro-Tak^{R}, wie Duro-TakR 326-1753, National Starch & Chemicals) in Ethylacetat und n-Hexan zugefügt und (gegebenenfalls mit weiteren Hilfsstoffen) homogen gemischt. Die Lösung wird mit Hilfe eines Ziehrakels auf eine silikonisierte Polyesterfolie (beispielsweise Gelroflex^{R}; Rexam Release) als Abziehfolie mit einer Naßschichtdicke von 450 *µ*m aufgetragen und bei 50 °C 1 h lang getrocknet.

Schicht B; diese Schicht wird analog Schicht A mit der einzigen Ausnahme hergestellt, daß die Tacrinbase durch die Selegilinbase ersetzt wird.

Danach wird die getrocknete Schicht A auf die zweite Schicht B auflaminiert. Von dem auf diese Weise erhaltenen Zweischicht-TTS wird die Abziehfolie der Selegilin-Schicht B abgezogen, wonach als Ersatz für die abgezogene Folie eine dünne Polyesterfolie (beispielsweise Hostaphan^{R}; Hoechst) als äußere Abdeckschicht (Backing-Folie) aufgebracht wird.

### Beispiel 3: Zweischicht-TTS mit einer die Wirkstoffabgabe steuernden Membran.

Analog Beispiel 2 werden zwei Schichten A und B hergestellt, wobei man aber zwischen diesen beiden Schichten eine die Wirkstoffpermeation steuernde Membran vorsieht (beispielsweise Cotran^{R} der Firma 3M; oder 1154P). Dieses Zweischicht-TTS ist wiederum so ausgebildet, daß es mit der Tacrin enthaltenden Schicht auf die Haut aufgeklebt werden kann.

### Beispiel 4: Zweibahniges TTS

Tacrinbase einerseits und Selegilinbase andererseits werden gemäß Beispiel 2 gelöst und mit dem Acrylatklebstoff gemischt. Danach werden zwei getrennte Bahnen nebeneinander auf eine Abziehfolie aufkaschiert, so daß eine Schicht Tacrin und die andere Schicht Selegilin enthält. Nach dem Trocknen der zweibahnigen Schicht und dem Auflaminieren einer Abdeckfolie werden TTS ausgestanzt, die je eine Bahn Tacrin und Selegilin aufweisen. Selbstverständlich sind die Anteile der beiden Wirkstoffe variabel einstellbar.

### Beispiel 5:

Für die Klebemittelschicht wurde ein druckempfindliches Klebemittel auf Silikon-Basis verwendet (trimethyliertes Siliciumdioxid, das mit Polydimethylsiloxan mit endständigen Trimethylsiloxy-Gruppen behandelt worden ist; Schichtdicke trocken (SD) etwa 35 bis 45 *µ*m; Flächengewicht (FG) etwa 50 bis 60 g/m²). Eine Abdeckfolie aus PET (Dicke etwa 75 *µ*m; FG etwa 100 g/m²) wurde mit dem Silikon-Kleber mit Hilfe einer Beschichtungsanlage beschichtet. Auf die beschichtete Abdeckfolie wurde eine mikroporöse Polypropylenmembran (heißsiegelfähig; Dicke etwa 50 *µ*m; FG etwa 120 g/m²) aufkaschiert, so daß ein Laminat aus Abdeckfolie, Klebemittel und Membran hergestellt wurde. Danach wurde das Laminat mit Hilfe einer Siegelmaschine (mit Schweißring) mit einer Trägerfolie aus Polyester (aluminiumbedampft) mit Polyolefin-Siegelschicht (heißsiegelfähig; Dicke etwa 70 *µ*m) derart verschweißt, daß ein Spalt zum Einfüllen einer Wirkstofflösung zurückblieb. Das befüllbare transdermale therapeutische System (Leer-TTS) wurde beispielsweise mit einer Hamilton-Spritze oder mit einer Schlauchpumpe mit Kanüle mit der folgenden Wirkstofflösung gefüllt:

Zusammensetzung der Wirkstofflösung pro TTS:

| | mg/TTS |
|---|---|
| Tacrin-Base | 40,0 |
| Selegilin-Base | 20,0 |
| Propylenglykol | 160,0 |
| α-Tocopherol | 80,0 |
| Insgesamt | 300,0 mg |
| Füllmenge des Reservoirs: | 300,0 mg |

Nach dem Befüllen wurde der Befüllungsspalt verschweißt. Befüllte transdermale therapeutische Systeme wurden mit Hilfe einer Stanze ausgestanzt.

## Patentansprüche

1. Pflaster zur transdermalen Applikation mit einer äußeren Abdeckschicht (backing layer), einer selbstklebenden Matrix (self-adhesive matrix) und einer entfernbaren Schutzschicht (release layer), wobei die Matrix,
- Tacrin und Selegilin (gegebenenfalls in Form ihrer pharmazeutisch verträglichen Salze) als Wirkstoff neben
- einem hydrophilen schwerflüchtigen Lösungsmittel und/oder
- einem lipophilen schwerflüchtigen Lösungsmittel enthält.

2. Pflaster nach Anspruch 1, **gekennzeichnet durch** einen Gehalt von bis zu 70 Gew.-% (bezogen auf das Matrixgewicht) an einem Lösungsmittel aus der **durch** hydrophile schwerflüchtige Lösungsmittel, lipophile schwerflüchtige Lösungsmittel und deren Gemische gebildeten Gruppe.

3. Pflaster nach Anspruch 1 oder 2, **dadurch gekennzeichnet, daß** die beiden Wirkstoffe im Gemisch in der Matrix vorliegen.

4. Pflaster nach einem der vorhergehenden Ansprüche, **dadurch gekennzeichnet, daß** die Matrix zwei aufeinanderfolgende Matrixschichten aufweist, wobei die eine Matrixschicht den einen und die andere Matrixschicht den anderen der beiden Wirkstoffe enthält.

5. Pflaster nach Anspruch 4, **dadurch gekennzeichnet, daß** die der Abdeckschicht (outer covering bzw. backing layer) zugewandte Matrixschicht Selegilin oder eins seiner Salze oder umgekehrt Tacrin oder eins seiner Salze als Wirkstoff enthält.

6. Pflaster nach Anspruch 4 oder 5, **dadurch gekennzeichnet, daß** die beiden Matrixschichten durch eine die Wirkstoffpermeation steuernde Membran getrennt sind.

7. Pflaster nach einem der Ansprüche 1 oder 2, **dadurch gekennzeichnet, daß** die Matrix zwei oder mehr aneinander angrenzende, nebeneinander in derselben Ebene angeordnete Zonen aufweist, die jeweils nur einen der beiden Wirkstoffe enthalten.

8. Pflaster nach Anspruch 7, **dadurch gekennzeichnet, daß** die Zonen bahnenartig ausgebildet sind.

9. Pflaster nach einem der vorhergehenden Ansprüche, **gekennzeichnet durch** einen Gehalt an Tacrin und an Selegilin (gegebenenfalls in Form ihrer Salze) von jeweils bis zu 50 Gew.-% (jeweils bezogen auf das Matrixgewicht).

10. Pflaster nach einem der vorhergehenden Ansprüche, **gekennzeichnet durch** eine Membran, insbesondere eine die Wirkstoffpermeation steuernde Membran.

11. Pflaster nach einem der vorhergehenden Ansprüche, **gekennzeichnet durch** eine Matrix auf Polyacrylatbasis, Silikon-basis oder Polyisobutylenbasis.

12. Pflaster nach Anspruch 10 und/oder 11, **gekennzeichnet durch** ein Klebeelement in Form einer die Membran vollständig oder an ihrer Peripherie ringförmig abdeckenden Schicht.

13. Pflaster nach Anspruch 10, 11 und/oder 12, **gekennzeichnet durch** ein Klebeelement aus einem druckempfindlichen Klebemittel auf Silikon-Basis.

14. Pflaster nach einem der vorhergehenden Ansprüche, **gekennzeichnet durch** einen Gehalt an α-Tocopherol oder α-Tocopherol-Derivat.

15. Pflaster nach einem der vorhergehenden Ansprüche, **gekennzeichnet durch** Propylenglykol, 1,2-Pentandiol, Glycerin, hydrophiles Cetiol oder Transcutol als hydrophiles schwerflüchtiges Lösungsmittel.

16. Pflaster nach einem der vorhergehenden Ansprüche, **gekennzeichnet durch** Copherol, Migliol, i-Propylmyristat oder lipophiles Cetiol als lipophiles schwerflüchtiges Lösungsmittel.

17. Pflaster nach einem der vorhergehenden Ansprüche, **gekennzeichnet durch** einen Gehalt an hydrophilem schwerflüchtigen Lösungsmittel und lipophilem schwerflüchtigen Lösungsmittel.

## Claims

1. Plaster for transdermal application with an outer backing layer, a self-adhesive matrix and a removable release layer, the matrix containing
- tacrine and selegiline (optionally in the form of their pharmaceutically compatible salts) as active substance in addition to
- a hydrophilic low volatility solvent and/or
- a lipophilic low volatility solvent.

2. Plaster according to claim 1 **characterised by** a content of up to 70 % by weight (based on the weight of the matrix) of a solvent from the group formed by hydrophilic low volatility solvents, lipophilic low volatility solvents and their mixtures.

3. Plaster according to claim 1 or 2 **characterised in that** the two active substances are present in the matrix in mixture.

4. Plaster according to one of the preceding claims **characterised in that** the matrix contains two matrix layers in sequence, one matrix layer containing one and the other matrix layer the other of the two active substances.

5. Plaster according to claim 4 **characterised in that** the matrix layer adjacent to the outer covering or backing layer contains selegiline or one of its salts or, conversely, tacrine or one of its salts as active substance.

6. Plaster according to claim 4 or 5 **characterised in that** the two matrix layers are separated by a membrane controlling the active substance permeation.

7. Plaster according to one of claims 1 or 2 **characterised in that** the matrix contains two or more adjacent zones arranged side by side in the same plane, each of which contains only one of the two active substances respectively.

8. Plaster according to claim 7 **characterised in that** the zones have the form of webs.

9. Plaster according to one of the preceding claims **characterised by** a content of tacrine (optionally in the form of its salts) of up to 50 % by weight and of selegiline (optionally in the form of its salts) of up to 50% by weight (based on the weight of the matrix each).

10. Plaster according to one of the preceding claims **characterised by** a membrane, in particular a membrane controlling the active substance permeation.

11. Plaster according to one of the preceding claims **characterised by** a matrix based on polyacrylate, silicone or polyisobuylene.

12. Plaster according to claim 10 and/or 11 **characterised by** an adhesive element in the form of a layer covering the membrane completely or only on its periphery in an annular manner.

13. Plaster according to claim 10, 11 and/or 12 **characterised by** an adhesive element of a pressure sensitive adhesive based on silicone.

14. Plaster according to one of the preceding claims **characterised by** a content of α-tocopherol or α-tocopherol derivative.

15. Plaster according to one of the preceding claims **characterised by** propylene glycol, 1,2-pentane diol, glycerine, hydrophilic Cetiol or Transcutol as hydrophilic low volatility solvent.

16. Plaster according to one of the preceding claims **characterised by** Copherol, Migliol, i-propyl myristate or lipophilic Cetiol as lipophilic low volatility solvent.

17. Plaster according to one of the preceding claims **characterised by** a content of hydrophilic low volatility solvent and lipophilic low volatility solvent.

## Revendications

1. Emplâtre pour l'application transdermique, comprenant une couche de recouvrement extérieure (backing layer), une matrice auto-adhésive (self-adhesive matrix) et une couche protectrice susceptible d'être enlevée (release layer), dans lequel la matrice contient :
- du Tacrin et du Selegilin (le cas échéant sous la forme de leurs sels pharmaceutiquement acceptables) à titre de principe actif, ainsi que
- un solvant hydrophile difficilement volatile et/ou
- un solvant lipophile difficilement volatil.

2. Emplâtre selon la revendication 1, **caractérisé par** une teneur allant jusqu'à 70 % en poids (par référence au poids de la matrice) d'un solvant choisi parmi le groupe des solvants hydrophiles difficilement volatils, les solvants lipophiles difficilement volatils, et leurs mélanges.

3. Emplâtre selon l'une ou l'autre des revendications 1 et 2, **caractérisé en ce que** les deux principes actifs sont présents dans la matrice en mélange.

4. Emplâtre selon l'une des revendications précédentes, **caractérisé en ce que** la matrice comprend deux couches de matrice successives, l'une des couches de matrice contenant l'un des principes actifs et l'autre couche de matrice contenant l'autre principe actif.

5. Emplâtre selon la revendication 4, **caractérisé en ce que** la couche de matrice tournée vers la couche de recouvrement (outer covering ou backing layer) contient du Selegilin ou l'un de ses sels, ou inversement du Tacrin ou l'un de ses sels, à titre de principe actif.

6. Emplâtre selon l'une ou l'autre des revendications 4 et 5, **caractérisé en ce que** les deux couches de matrice sont séparées par une membrane qui commande la perméation des principes actifs.

7. Emplâtre selon l'une ou l'autre des revendications 1 et 2, **caractérisé en ce que** la matrice comporte deux ou plusieurs zones mutuellement limitrophes, agencées les unes à côté des autres dans le même plan, qui contiennent chacune un seul des deux principes actifs.

8. Emplâtre selon la revendication 7, **caractérisé en ce que** les zones sont réalisées à la manière de bandes.

9. Emplâtre selon l'une des revendications précédentes, **caractérisé par** une teneur de Tacrin et de Selegilin (le cas échéant sous la forme de leurs sels) allant respectivement jusqu'à 50 % en poids (en se référant au poids de matrice).

10. Emplâtre selon l'une des revendications précédentes, **caractérisé par** une membrane, en particulier par une membrane qui commande la perméation des principes actifs.

11. Emplâtre selon l'une des revendications précédentes, **caractérisé par** une matrice à base de polyacrylate, à base de silicone, ou à base de polyisobutylène.

12. Emplâtre selon l'une ou l'autre des revendications 10 et/ou 11, **caractérisé par** un élément collant sous la forme d'une couche qui recouvre la membrane, soit complètement, soit sous forme d'un anneau à sa périphérie.

13. Emplâtre selon l'une des revendications 10, 11 et/ou 12, **caractérisé par** un élément collant formé par un adhésif sensible à la pression à base de silicone.

14. Emplâtre selon l'une des revendications précédentes, **caractérisé par** une teneur de α-Tocophérol, ou de dérivé de α-Tocophérol.

15. Emplâtre selon l'une des revendications précédentes, **caractérisé par** du propylène-glycol, 1,2-pentanediol, glycérine, cétiol ou transcutol hydrophile, à titre de solvant hydrophile difficilement volatil.

16. Emplâtre selon l'une des revendications précédentes, **caractérisé par** du cophérol, migliol, i-propylmyristate, ou du cétiol lipophile, à titre de solvant lipophile difficilement volatil.

17. Emplâtre selon l'une des revendications précédentes, **caractérisé par** une teneur de solvant hydrophile difficilement volatil et de solvant lipophile difficilement volatil.
